# EUROPEAN PATENT APPLICATION

(11) **EP 3 949 851 A1**
(43) Date of publication of application: **09.02.2022**
(21) Application number: 20776918.3
(22) Date of filing: 27.03.2020
(51) Int. Cl.: A61B 5/05

(54) **BODY COMPOSITION MONITOR, BODY COMPOSITION MONITORING PROGRAM, AND COMPUTER-READABLE NON-TRANSITORY STORAGE MEDIUM**

(30) Priority: 28.03.2019 JP 2019063487
(71) Applicant: Tanita Corporation, Tokyo 174-8630 (JP)
(72) Inventor: NAGAHAMA Toshiki, Tokyo 174-8630 (JP); KASAHARA Yasuhiro, Tokyo 174-8630 (JP)
(74) Representative: Eisenführ Speiser
(86) International application number: PCT/JP2020/013917
(87) International publication number: WO 2020/196813

(57) **Abstract**

The present disclosure provides a body composition analyzer and a body composition measurement program capable of determining a measurement abnormality by analyzing a waveform of Dynamic Impedance (DI) . A body composition analyzer (10) that measures body composition based on the measurement of bioelectrical impedance includes a bioelectrical impedance measuring unit (112) that acquires time-series data of the bioelectrical impedance by measurement, and a measurement abnormality determination unit (116) that determines a cause or type of an abnormality in the measurement based on the time-series data.

## Description

### CROSS-REFERENCE TO RELATED APPLICATIONS

This application claims the benefit of Patent Application No. 2019-063487 filed in Japan on March 28, 2019, the contents of which application are hereby incorporated by reference.

### TECHNICAL FIELD

The present disclosure relates to a body composition analyzer for measuring body composition based on measurement of bioelectrical impedance, a body composition measurement program, and a computer-readable non-transitory storage medium recording the program.

### BACKGROUND TECHNOLOGY

Conventionally, body composition analyzers are known to measure body composition based on information such as height, weight, age, and gender, and bioelectrical impedance of each part of the human body obtained by measurement.

In order to successfully measure the body composition based on the bioelectrical impedance, it is assumed that the bioelectrical impedance is measured normally. A device having a circuit for detecting abnormalities in the measurement of bioelectrical impedance is disclosed in JP2011079574A, which appropriately detects an abnormality when the contact impedance increases due to a decrease in the contact area of the sole, which is a contact area between a human body and an electrode.

In addition, JP5110277B also discloses a device having a circuit for detecting abnormal bioelectrical impedance measurement, which detects an abnormal value using a specific judgment formula from the measured values of bioelectrical impedance.

### SUMMARY

However, if a circuit for detecting measurement abnormality is provided separately from a circuit for obtaining bioelectrical impedance, the circuit configuration of the body composition analyzer becomes complicated.

One of the purposes of the present disclosure is to provide a body composition analyzer and a body composition measurement program that can determine a measurement abnormality without changing the circuit configuration of an existing body composition analyzer by performing waveform analysis of Dynamic Impedance (DI).

The present disclosure adopts the following technical solutions to solve the above problem. The signs in parentheses in the claims and this section are examples showing the correspondence with the specific means described in the embodiments described below as one form, and do not limit the technical scope of the present disclosure.

A body composition analyzer in one aspect is a body composition analyzer for measuring body composition based on the measurement of bioelectrical impedance, comprising: a bioelectrical impedance measuring unit configured to acquire time-series data of bioelectrical impedance by measurement, and a measurement abnormality determination unit configured to determine a cause or type of abnormality in the measurement based on the time-series data.

This configuration enables the cause or type of measurement abnormality to be determined based on the time-series change of the measured bioelectrical impedance. Therefore, the measurement abnormality can be determined without having a circuit for detecting the measurement abnormality separately from the circuit for measuring bioelectrical impedance.

The measurement abnormality determining unit may be configured to determine the cause or type of the abnormality of the measurement based on a trend of the time-series data.

With this configuration, for example, a measurement abnormality can be determined using a trend (trend variation) such as a slope of a linear function (SL) when the time-series data is approximated by a linear function.

The measurement abnormality determination unit may be configured to determine the cause or type of the abnormality of the measurement based on the variation of the time-series data.

With this configuration, for example, measurement abnormality can be determined using the variation of standard deviation (SD), variance, unbiased variance, etc. of the time-series data.

The bioelectrical impedance may include resistance.

With this configuration, measurement abnormalities can be determined based on the resistance, which is mainly electrically derived from the extracellular fluid.

The bioelectrical impedance may include reactance.

This configuration allows more accurate determination of measurement abnormalities based on reactance, which is mainly derived electrically from intracellular fluid and cell membranes.

The system may further comprise a notification unit configured to notify a remedial measure corresponding to the cause or type of the abnormality in the measurement.

This configuration allows the user to know the cause of the measurement abnormality and to know the improvement measures for normal measurement corresponding to the type of measurement abnormality.

A body composition measurement program in one aspect is a body composition measurement program for controlling a body composition analyzer equipped with a computer for measuring body composition based on measurement of bioelectrical impedance, the program causing the computer to: acquire time-series data of the bioelectrical impedance by measurement; and determine a cause or type of abnormality in the measurement based on the time-series data.

### BRIEF DESCRIPTION OF THE DRAWINGS

Fig. 1 shows a body composition analyzer of the first embodiment;
Fig. 2 shows a block diagram of the body composition analyzer of the first embodiment;
Fig. 3A shows a time-series waveform of resistance (R) for a normal DI of the first embodiment;
Fig. 3B shows a time-series waveform of reactance (X) for a normal DI of the first embodiment;
Fig. 3C shows time-series waveforms of R and X for a normal DI of the first embodiment;
Fig. 4A shows a time-series waveform of R for the DI of the first embodiment in a situation of electrode non-contact;
Fig. 4B shows a time-series waveform of X for the DI of the first embodiment in a situation of electrode non-contact;
Fig. 4C shows time-series waveforms of R and X for the DI of the first embodiment in a situation of electrode non-contact;
Fig. 5A shows a time-series waveform of R for DI of the first embodiment in a situation of drying;
Fig. 5B shows a time-series waveform of X for DI of the first embodiment in a situation of drying;
Fig. 5C shows a trajectory of time-series data between R and X for DI of the first embodiment in a situation of drying;
Fig. 6A shows a time-series waveform of R for DI of the first embodiment in a situation of body movement;
Fig. 6B shows a time-series waveform of X for DI of the first embodiment in a situation of body movement;
Fig. 6C shows a trajectory of time-series data between R and X for DI of the first embodiment in a situation of body movement;
Fig. 7 shows a table of the first embodiment, which indicates the relationship between the following (a) and (b): (a) the absolute value (|R_{SL}|) of the slope of the linear function and the standard deviation (R_{SD}) when the time-series data of R is approximated by the linear function; and (b) the situations of electrode non-contact, drying, and body movement;
Fig. 8 shows an example of the first embodiment which informs the user of remedial measures corresponding to the cause and type of a measurement abnormality;
Fig. 9 shows a flow chart of the first embodiment, for determining a measurement abnormality based on the absolute value (|R_{SL}|) of the slope of the linear function and the standard deviation (R_{SD}) when the time-series data of R is approximated by a linear function;
Fig. 10 shows a relationship of the second embodiment between the following (a) and (b): (a)the absolute value (|R_{SL}|) of the slope of the linear function and the standard deviation (R_{SD}) when the time-series data of R is approximated by a linear function and the absolute value (|X_{SL}|) of the slope of the linear function and the standard deviation (X_{SD}) when the time-series data of X is approximated by a linear function; and (b) the situations of electrode non-contact, drying, and body movement; and
Fig. 11 shows a flow chart of the second embodiment, for determining a measurement abnormality based on the absolute value (|R_{SL}|) of the slope of the linear function and the standard deviation (R_{SD}) when the time-series data of R is approximated by a linear function and the absolute value (|X_{SL}|) of the slope and the standard deviation (X_{SD}) of the linear function when the time-series data of X is approximated by a linear function.

### DESCRIPTION OF THE PREFERRED EMBODIMENTS

The following is a description of embodiment of the present disclosure with reference to the drawings. The embodiment described below show an example of how to implement the present disclosure, and does not limit the present disclosure to the specific configuration described below. In the implementation of the present disclosure, the specific configuration according to the embodiment may be adopted as appropriate.

### (First Embodiment)

### [Configuration of the body composition analyzer 10]

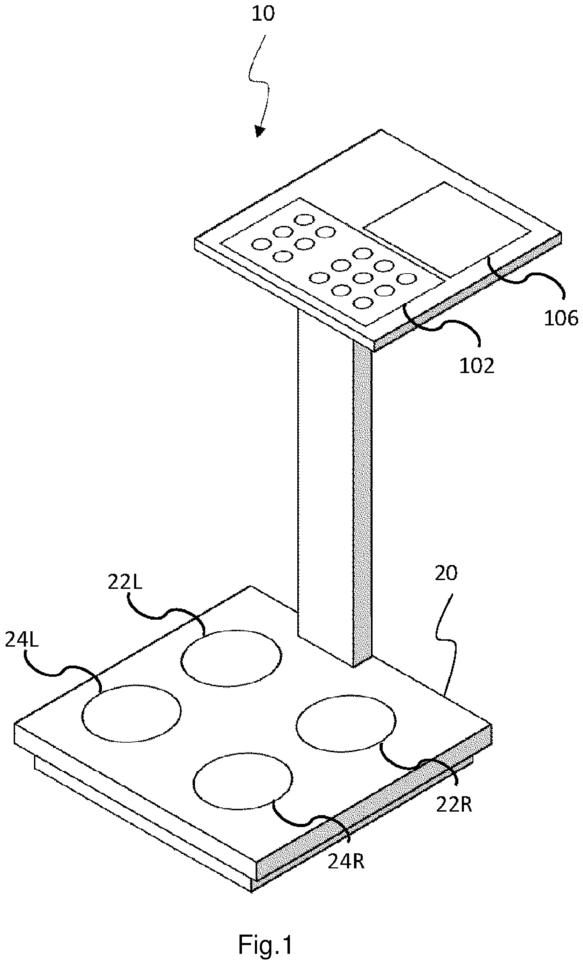

Fig. 1 shows a body composition analyzer 10 of the first embodiment. The body composition analyzer 10 can measure body weight and body composition as biometric information. The body composition analyzer 10 is equipped with a main unit 20, an input unit 102, and a display unit 106.

The main unit 20 is equipped with a load cell inside for measuring the weight, and can measure the weight of a user.

The main body 20 is equipped with a current-carrying electrode 22L and a measuring electrode 24L on the left side of the top surface, and a current carrying electrode 22R and a measuring electrode 24R on the right side of the top surface. The user stands upright with bare feet on top of the main unit 20 to take biometric measurements. At this time, the base of the left toe comes in contact with the current-carrying electrode 22L, the heel of the left foot comes in contact with the measuring electrode 24L, the base of the right toe comes in contact with the current-carrying electrode 22R, and the heel of the right foot comes in contact with the measuring electrode 24R.

The input unit 102 is an input means for inputting data into the body composition analyzer 10. The method of inputting information by the input unit 102 may be, for example, a manual method, a method via a recording medium, a method via wired communication, a method via wireless communication, or any other method.

The manual input method may be, for example, a button type, a dial type, or a touch sensor type. The recording medium of the method via a recording medium may be, for example, flash memory, CD-ROM, or DVD-ROM. The wireless communication of the method via wireless communication may be, for example, the Internet, a wireless LAN such as Wi-Fi (registered trademark), or a short-range wireless communication such as Bluetooth (registered trademark) or NFC (Near Field Communication) . In this embodiment, the input unit 102 is a manual input method and is a button type.

The user operates the input unit 102 to input data such as the user's height, age, and gender to the body composition analyzer 10. The body composition analyzer 10 calculates the body composition data by combining this data with the body weight and the bioelectrical impedance. The body composition data includes, for example, body fat percentage, body fat mass, muscle mass, abdominal/back muscle ratio, body water content, bone mass, visceral fat area, and basal metabolism.

The output unit 106 is an output means for outputting the measurement results of the body composition analyzer 10. The measurement results are, for example, body weight, body composition data, and the like. The output unit 106 is, for example, a display panel equipped with an LCD (Liquid Crystal Display) or an OLED (Organic Light Emitting Diode). The output unit 106 may be integrated with the body composition analyzer 10, or may not be integrated with the body composition analyzer 10, such as a smartphone or tablet. In the present embodiment, the output unit 106 is a display panel equipped with an LCD integrated with the body composition analyzer 10.

The output unit 106 may, for example, display numerical values reflecting the results of the user's measurement, text, a diagram of the user's standing position at the time of measurement, or the like, or may output the data in audio or other formats. The output unit 106 may also display remedial measures corresponding to the cause or type of measurement abnormality reported to the user by a notification unit 118 described below.

### [Functional Configuration of Body Composition Analyzer 10]

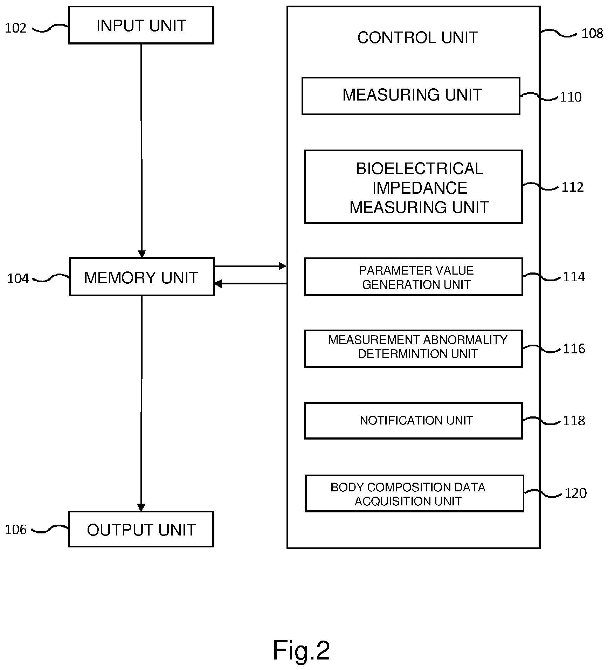

Fig. 2 shows a block diagram of the body composition analyzer 10 of the first embodiment. The body composition analyzer 10 has an input unit 102, a memory unit 104, an output unit 106, and a control unit 108.

The memory unit 104 is a memory that can store data. The memory can be, for example, volatile memory (e.g., RAM (Random Access Memory)), non-volatile memory (e.g., ROM (Read Only Memory)), etc. The memory unit 104 may be built into the body composition analyzer 10 or may be provided outside the body composition analyzer 10, such as an external hard disk drive as shown in Fig. 2, an external server, or the like. In the present embodiment, the memory unit 104 is built into the body composition analyzer 10.

The memory unit 104 stores a program executed by the control unit 108, data input to the body composition analyzer 10 by a user operating the input unit 102, statistical data for calculating body composition data by the body composition analyzer 10, body composition data calculated by the body composition analyzer 10, and the like. The program may be provided to the body composition analyzer 10 by the body composition analyzer 10 by downloading it from a communication network, or may be provided to the body composition analyzer 10 via a non-transitory storage medium.

The control unit 108 is a control device that controls the input unit 102, the memory unit 104, the output unit 106, the weight measuring unit 110, the bioelectrical impedance measuring unit 112, the parameter value generation unit 114, the measurement abnormality determination unit 116, the notification unit 118, and the body composition data acquisition unit 120. The control unit 108 is equipped with a central processing unit (CPU). The control unit 108 is connected to each unit via electric communications. The control unit 108 realizes the functions of each unit by executing a program stored in the memory unit 104.

The weight measuring unit 110 is a weight measuring means for measuring the weight of a user. The weight measuring unit 110 measures the weight using the load cell described above. Specifically, the load cell consists of a straining body of a metal member that deforms in response to a load, and a strain gauge that is affixed to the straining body. When a user rides on top of the body composition analyzer 10, the load of the user causes the load cell's straining body to bend and the strain gauge to expand or contract. The resistance value (output value) of the strain gauge changes in accordance with the expansion or contraction. The weight measuring unit 110 calculates the weight from the difference between the output value of the load cell when no load is applied (zero point) and the output value when a load is applied. The weight measuring unit 110 calculates the body weight from the difference between the output value of the load cell when no load is applied (zero point) and the output value when a load is applied. The same configuration for measuring the body weight using the load cell as that of general scales can be used.

The bioelectrical impedance measuring unit 112 is a measurement means to acquire time-series data of bioelectrical impedance by measurement. Bioelectrical impedance is an electrical resistance value obtained by passing a weak electric current through the body and measuring the ease with which this current flows. The bioelectrical impedance measuring unit 112 passes a weak electric current through the body and measures it via the current-carrying electrode 22L and measuring electrode 24L shown in Fig. 1.

The bioelectrical impedance is obtained from the measured current and voltage. The bioelectrical impedance includes a resistance component (resistance: R), which is mainly derived electrically from the extracellular fluid, and a capacitance component (reactance: X), which is mainly derived electrically from the intracellular fluid and cell membrane. By examining the time-series data of R and X, it is possible to determine whether the measurement is normal or abnormal, and if the measurement is abnormal, the cause or type of the abnormality. The R and X used for this determination can be R and X obtained by applying a current of a certain frequency, or the R and X obtained by applying a current of multiple frequencies. In this embodiment, R and X are R and X obtained by passing a current of a certain frequency.

In particular, when determining the cause or type of measurement abnormality by considering the reactance X, a current with a lower frequency may be used as compared to a current with a higher frequency. Among them, especially when the cause is when the electrode is not in contact and when it is dry, a current with a lower frequency may be used compared to a current with a higher frequency. The reason for this is that the lower the frequency of the current, the greater the effect of the electrical capacitance of the capacitor (the smaller the ω in X = 1 / jωC, the greater the effect of C on the size of X) . A current with a low frequency is, for example, a current with a frequency of 50 kHz or lower.

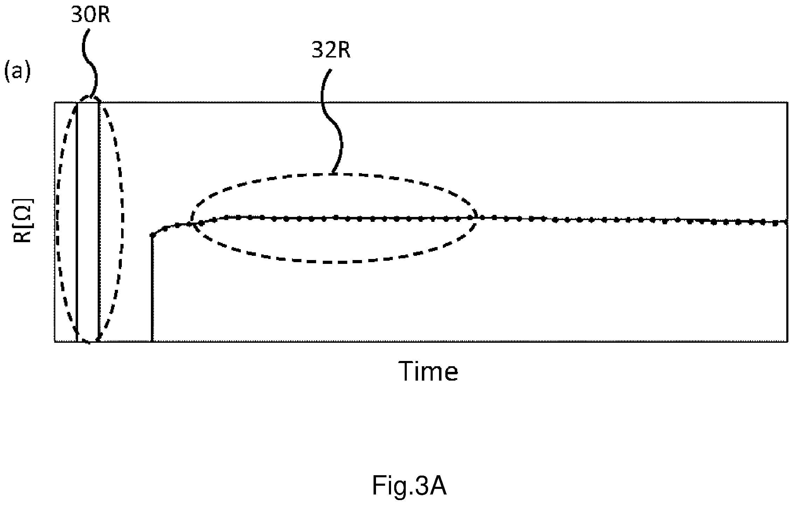

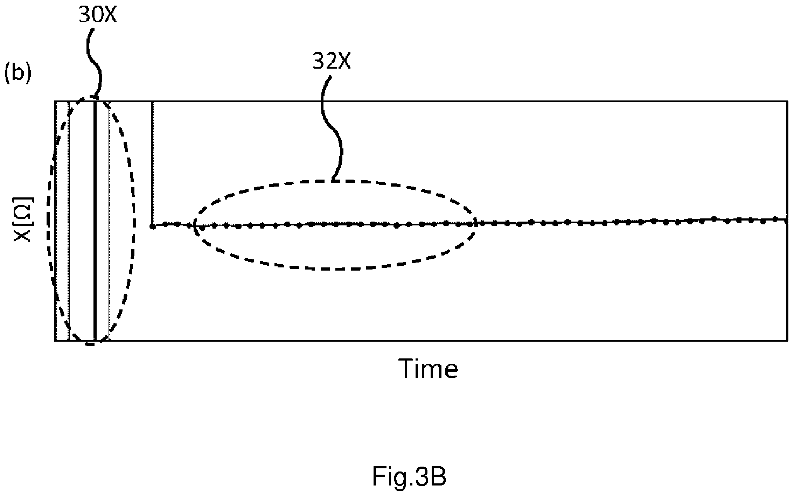

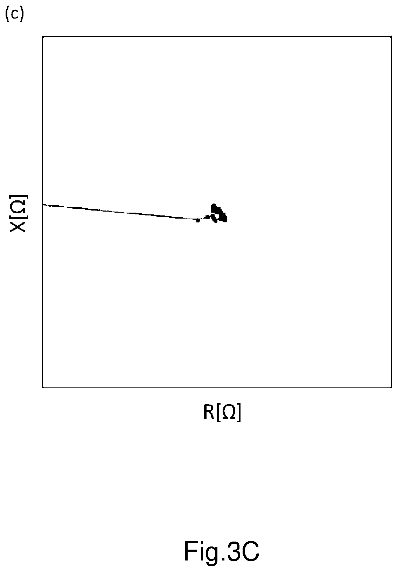

First, the time-series data of R and X when the measurement is normal will be explained. Fig. 3A shows a time-series waveform of resistance (R) for a normal DI of the first embodiment, Fig. 3B shows a time-series waveform of reactance (X) for a normal DI of the first embodiment, and Fig. 3C shows time-series waveforms of R and X for a normal DI of the first embodiment.

As shown in Figs. 3A and 3B, the time-series waveforms of R and X do not have fixed values when the user does not touch the electrodes (30R, 30X), but the values stabilize as soon as the user starts riding the body composition analyzer 10 (32R, 32X). As a result, as shown in Fig. 3C, the trajectory of the time-series data with R and X is a set of points that gather almost in one place.

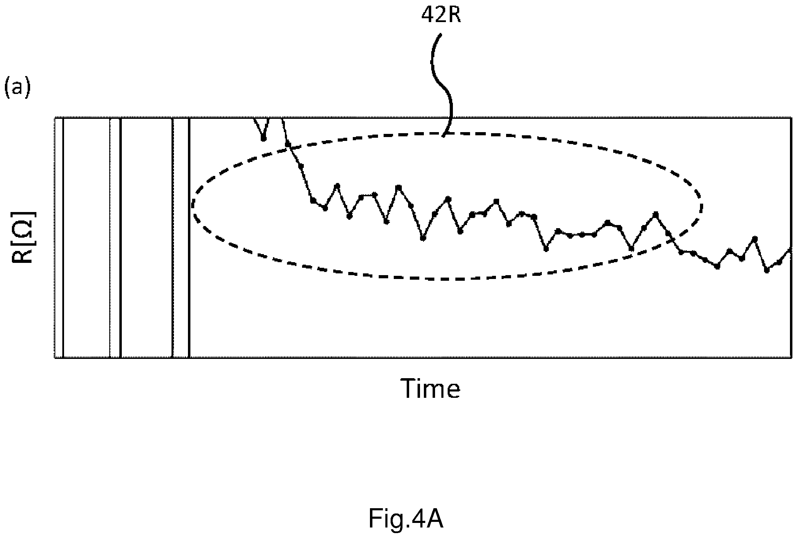

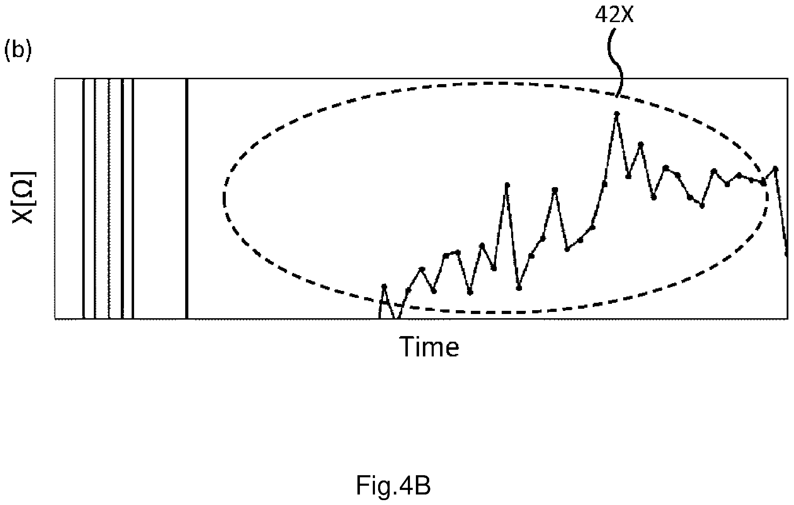

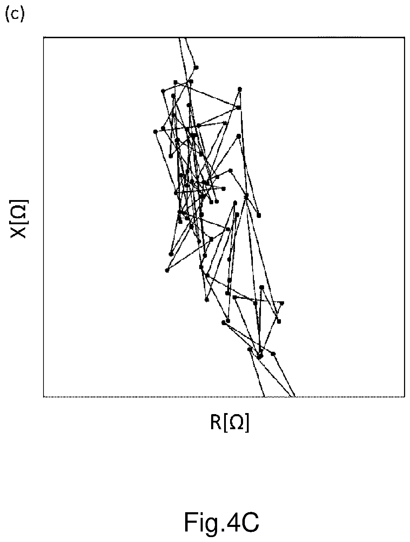

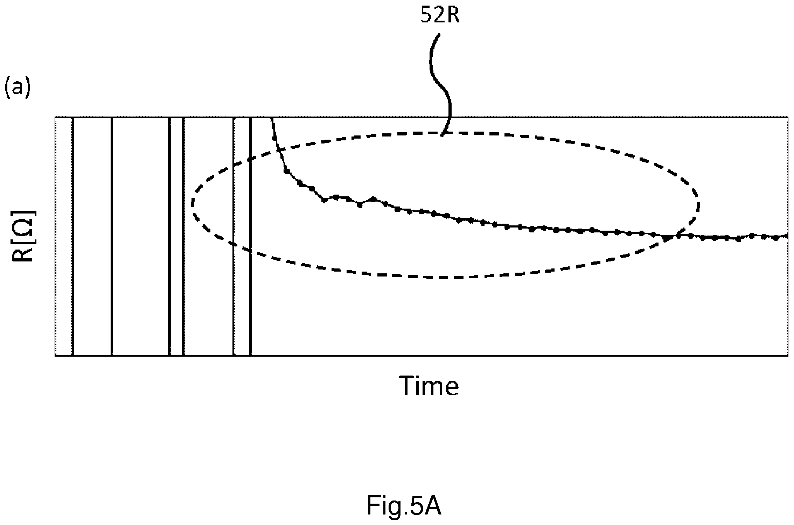

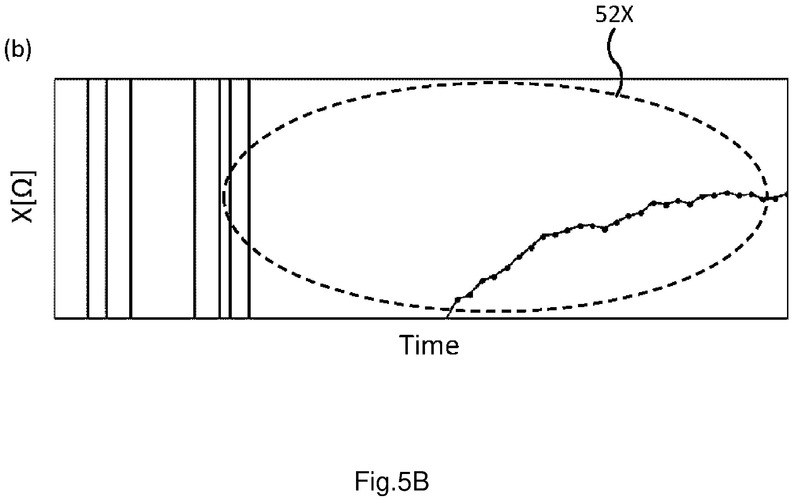

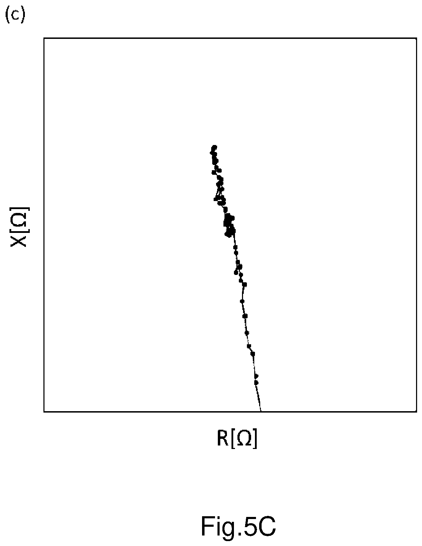

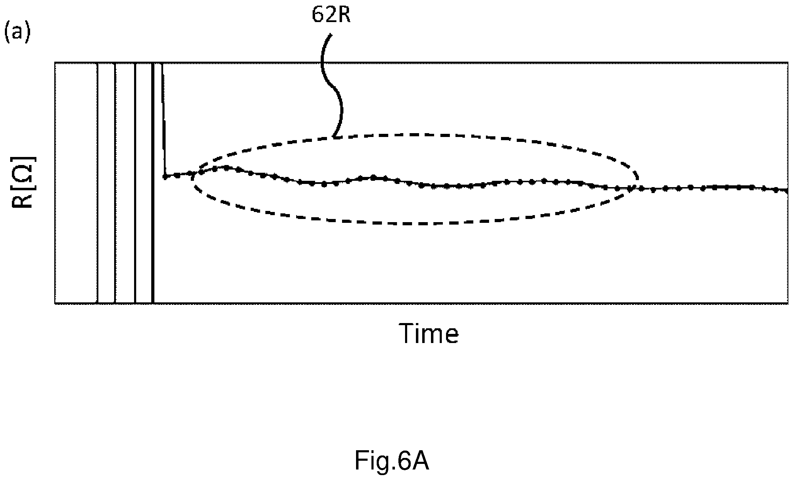

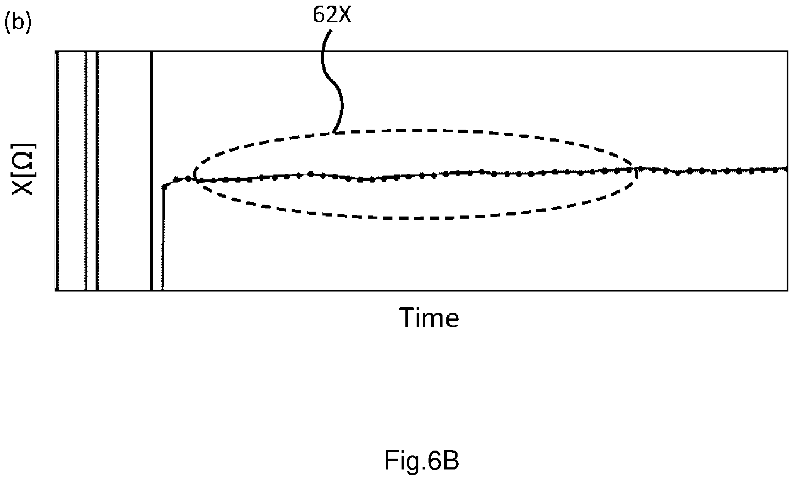

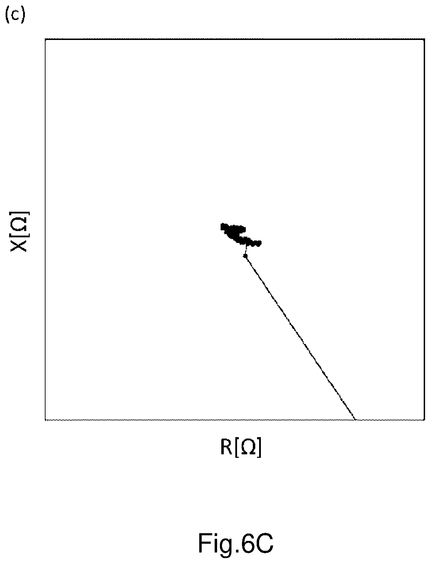

Next, the time-series data of R and X when the measurement is abnormal will be explained. A situation of abnormal measurement includes, for example, a situation in which the electrodes and the body are not in proper contact as shown in Fig. 4 (hereinafter also referred to as "situation of electrode non-contact"), a situation in which the skin is dry or socks are worn as shown in Fig. 5 (hereinafter also referred to as "situation of drying"), a situation in which there is body movement as shown in Fig. 6 (hereinafter also referred to as "situation of body movement"), and so on.

In the situation of electrode non-contact, the time-series data is as shown in Fig. 4. Fig. 4A shows a time-series waveform of R for the DI of the first embodiment in a situation of electrode non-contact, Fig. 4B shows a time-series waveform of X for the DI of the first embodiment a situation of electrode non-contact, and Fig. 4C shows time-series waveforms of R and X for the DI of the first embodiment a situation of electrode non-contact.

In the situation of electrode non-contact, the current and voltage values are undefined. Therefore, compared to the normal measurement in Fig. 3, the values of R and X are not stable (42R, 42X) even after the user gets on as shown in Fig. 4A and 4B. As a result, the trajectory of the time-series data of R and X becomes a set of points that are scattered throughout, as shown in Fig. 4C.

Next, the time-series data between R and X in the situation of drying becomes the time-series data as shown in Fig. 5. Fig. 5A shows a time-series waveform of R for DI of the first embodiment in the situation of drying, Fig. 5B shows a time-series waveform of X for DI of the first embodiment in the situation of drying, and Fig. 5C shows a trajectory of time-series data between R and X for DI of the first embodiment in the situation of drying.

In the situation of drying, the air layer between the living body and the electrodes is increased compared to the normal measurement in Fig. 3. As the user rides the body composition analyzer 10 and time passes, the value of R and the value of X stabilize because sweat and other factors gradually increase the adhesion with the electrodes.

In particular, in the situation of drying, the thickness of the air layer between the living body and the electrodes is thick, so the contact resistance between the skin and the electrodes is large and the electric capacitance of the capacitor is small. As time passes, the thickness of the air layer becomes thinner, the contact resistance between the skin and the electrodes becomes smaller, and the electrical capacitance of the capacitor becomes larger. As the electrical capacitance of the capacitor increases, the reactance (X) increases. In other words, as the user rides the body composition analyzer 10 and time passes, the electrical capacitance of the capacitor gradually increases and the reactance (X) gradually increases.

Therefore, as shown in Figs. 5A and 5B, the values gradually stabilize (52R, 52X) as time passes after the user rides on the body composition analyzer 10, compared to the normal measurement in Fig. 3. In addition, X becomes gradually larger (52X) as time passes since the user rode the body composition analyzer 10. As a result, the trajectory of the time-series data of R and X become a set of points spread out in the X direction, as shown in Fig. 5C.

Next, the time-series data of R and X in the situation of body movement will be explained. Fig. 6A shows a time-series waveform of R for DI of the first embodiment in the situation of body movement, Fig. 6B shows a time-series waveform of X for DI of the first embodiment in the situation of body movement, and Fig. 6C shows a trajectory of time-series data between R and X for DI of the first embodiment in the situation of body movement.

In the situation of body movement, the muscle cross-sectional area and muscle length of the measurement site change. Since muscle cross-sectional area is related to resistance (R) and muscle length is related to reactance (X), when the muscle cross-sectional area and muscle length of the measurement site change, both resistance (R) and reactance (X) change. However, the changes are smaller than when the electrodes are not in contact.

Therefore, as shown in Figs. 6A and 6B, compared to the normal measurement in Fig. 3, the values of R and X change a little (62R, 62X) even after the user starts riding the body composition analyzer 10. As a result, as shown in Fig. 6C, the trajectory of the time-series data of R and X becomes a set of points slightly spread out from one place.

Return to Fig. 2 to continue the explanation. The parameter value generation unit 114 generates values of parameters for time-series changes based on the time-series data of R shown in Figs. 4 to 6 above. In this embodiment, the parameter value generation unit 114 generates the value of the absolute value (|R_{SL}|) of the slope of the linear function and the value of the standard deviation (R_{SD}) when the time-series data of R is approximated by a linear function as the value of the parameter pertaining to the time-series change.

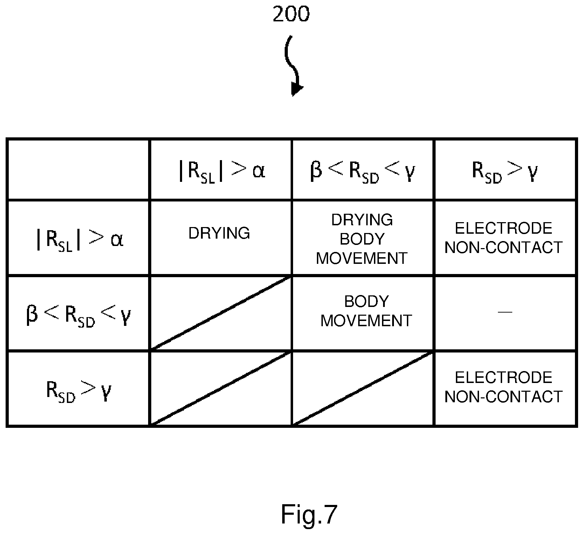

Fig. 7 shows a table of the first embodiment, which indicates the relationship between the following (a) and (b): (a) the absolute value (|R_{SL}|) of the slope of the linear function and the standard deviation (R_{SD}) when the time-series data of R is approximated by the linear function, and (b) the situations of electrode non-contact, drying, and body movement. The measurement abnormality determination unit 116 is equipped with the table 200 shown in Fig. 7. The α, β, and γ in the table 200 shown in Fig. 7 are threshold values determined from experimental data as appropriate. The measurement abnormality determination unit 116 refers to this table to determine whether the measurement is abnormal or not and the type of abnormality when the measurement is abnormal based on the value of the parameter | R_{SL} | generated by the parameter value generation unit 114 and the value of R_{SD}. The following is a specific explanation of how the parameters | R_{SL} | and R_{SD} characterize the time-series data of R.

First of all, in the situation of electrode non-contact, the variation in the value of R is larger than that of normal measurement. Therefore, whether or not the electrode and the living body are in correct contact is evaluated by a parameter that reflects the variation in the value of R. This parameter is, for example, the standard deviation, variance, and unbiased variance. This parameter can be, for example, a parameter based on the data obtained by offsetting the time-series data of R with an approximation function to be described later. In this embodiment, this parameter is the standard deviation (R_{SD}) based on the data obtained by offsetting the time-series data of R with the approximation function described below.

Therefore, the parameter value generation unit 114 generates the value of R_{SD} from the time-series data of R. The measurement abnormality determination unit 116 determines that the situation corresponds to "R_{SD} > γ" in the table 200 and the electrode and the living body are not in correct contact when the value of R_{SD} generated by the parameter value generation unit 114 is greater than γ (hereinafter, this determination is also referred to as "electrode non-contact").

In the situation of drying, the value of R gradually stabilizes as time passes, compared to normal measurement. Therefore, whether the skin is dry or not, or whether the person is wearing socks or not, is evaluated by a parameter that reflects the trend (trend variation) of the value of R. This parameter may be obtained, for example, from an approximation function of the time-series data of R. The approximation method to obtain the approximation function is, for example, the maximum likelihood estimation method, the least-squares method, etc. In the case where the approximate function is obtained by the least-squares method, this parameter may be, for example, the absolute value of the slope of the linear function when the linear function is approximated, or the coefficient of the variable when the exponential function is approximated. In this embodiment, this parameter is the absolute value (|R_{SL}|) of the slope of the linear function when the time-series data of R is approximated by the linear function based on the least-squares method.

Therefore, the parameter value generation unit 114 generates the value of "|R_{SL}|" from the time-series data of R. The measurement abnormality determination unit 116 determines that the situation corresponds to "|R_{SL}| > α" in the table 200 and the skin is dry or the use is wearing socks when the value of "|R_{SL}|" generated by the parameter value generation unit 114 is greater than α (hereinafter, this determination is also referred to as "drying").

In the situation of body movement, there is a variation in the value of R compared to normal measurement. Therefore, as in the situation of electrode non-contact, whether there is body movement or not is evaluated by the standard deviation. However, the variation in the value of R in the situation of body movement is smaller than that in the situation of electrode non-contact.

Therefore, the parameter value generation unit 114 generates the value of R_{SD} from the time-series data of R. When the value of R_{SD} generated by the parameter value generation unit 114 is smaller than γ but larger than β, the measurement abnormality determination unit 116 determines that the situation corresponds to "γ > R_{SL} > β" in the table 200 and there is body movement (hereinafter, this determination is also referred to as "body movement").

When multiple types of abnormality are determined, the type of abnormality may be determined according to the priority order of "electrode non-contact," "drying," and "body movement." For example, when "|R_{SL}| > α" and "R_{SD} > γ", "non-contact electrode" may be determined as shown in Fig. 7.

Returning to Fig. 2, the explanation continues. The notification unit 118 is an informing means for informing the user of remedial measures corresponding to the cause or type of the measurement abnormality when the measurement abnormality determination unit 116 determines that a measurement abnormality has occurred. The notification unit 118 informs the user via the output of the display unit 106.

The "type" of the measurement abnormality is expressed as a combination of the parameters and determination results shown in Fig. 7 above, the "cause" of the measurement abnormality is the type of this measurement abnormality expressed in the form of a cause, and the "remedial measure" corresponding to the type of the measurement abnormality is a remedial measure to eliminate any "type" of the measurement abnormality. The "remedial measure" corresponding to the type of measurement abnormality refers to a method to eliminate what is determined to be one of the "types" of measurement abnormalities. For example, the combination of "X_{SD} > γ" and "electrode non-contact" shown in Fig. 7 is the "type" of measurement abnormality, and the "cause" of the measurement abnormality is the type of measurement abnormality expressed in the form of the cause "The leg is not placed on the left leg electrode." The "remedial measure" is to "Place the left leg correctly on the left leg electrode" in order to eliminate the determination of "electrode non-contact" among the measurement abnormalities.

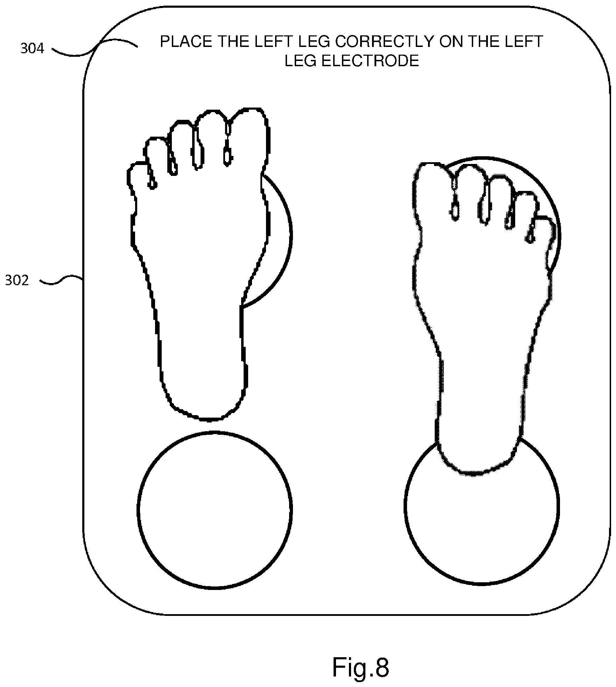

Fig. 8 illustrates this notification in detail. Fig. 8 shows an example of the first embodiment which informs the user of remedial measures corresponding to the cause and type of a measurement abnormality. As shown in 302 in Fig. 8, the notification unit 118 notifies the cause or type of the measurement abnormality to the user by graphically displaying the state in which the left leg of the user is not touching the electrode. For example, when the notification unit 118 notifies the user that the left leg is not touching the electrodes, it may inform the user of the measurement abnormality by lighting an LED lamp or the like, displaying "The left leg is not on the electrodes," or outputting an audio message "The left leg is not on the electrodes."

In addition, the notification unit 118 may notify the user of remedial measures according to the cause or type of the measurement abnormality. As shown in 304 in Fig. 8, the notification unit 118 may display "Place the left leg electrode correctly" in the situation of electrode non-contact. The notification unit 118 may, for example, notify "Please wet your skin or take off your socks" in the situation of drying, and "Please do not move and remain stationary" in the situation of body movement.

Returning to Fig. 2, the explanation continues. The body composition data acquisition unit 120 is a means of acquiring the body composition data of the user. The body composition data acquisition unit 120 obtains body composition data, such as body fat percentage, from the bioelectrical impedance, height, weight, age, gender, etc., by using, for example, the Bioelectrical Impedance Analysis (BIA) and multiple regression analysis.

### [Operation flow of the body composition analyzer 10]

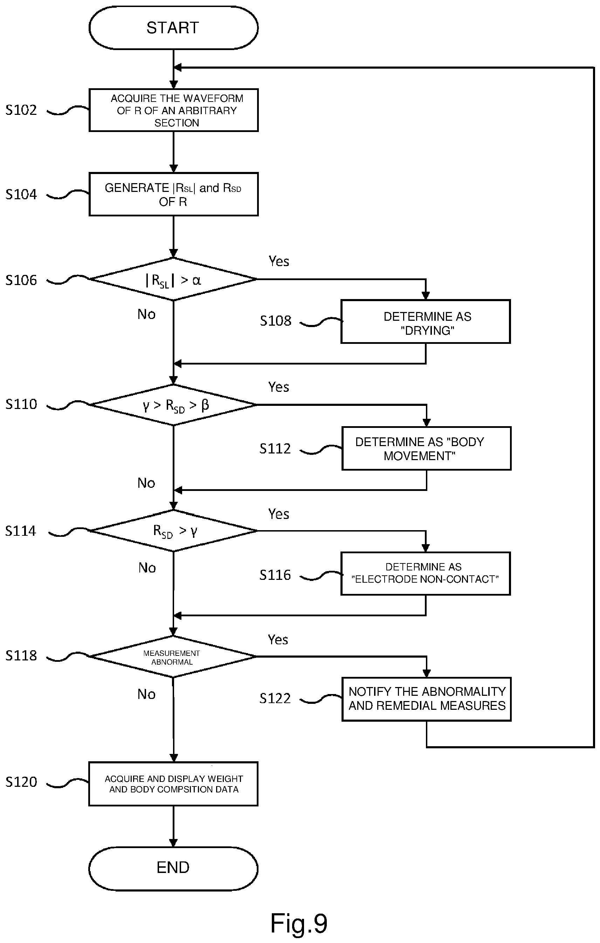

The following describes a flow for realizing the operation of the body composition analyzer 10 of the first embodiment by the above-described configuration of the body composition analyzer 10. Fig. 9 shows a flow chart of the first embodiment, for determining a measurement abnormality based on the absolute value (|R_{SL}|) of the slope of the linear function and the standard deviation (R_{SD}) when the time-series data of R is approximated by a linear function. The flow starts when the user is on the body composition analyzer 10.

First, the body composition analyzer 10 acquires the waveform of resistance (R) of an arbitrary section (step S102).

When the body composition analyzer 10 acquires the waveform of R of an arbitrary section, it generates the values of |R_{SL}| and R_{SD} (Step S104). Then, the body composition analyzer 10 determines whether these values are "|R_{SL}| > α" (Step S106), "γ > R_{SD} > β" (Step S110), and "R_{SD} > γ" (Step S114), respectively.

First, when it is determined that the value of "|R_{SL}|" is "|R_{SL}| > α" and "drying" (Step S106: Yes, S108) or not "|R_{SL}| > α" (Step S106: No), it proceeds to the step of determining whether "γ > R_{SD} > β" or not.

Next, if it is determined that the value of R_{SD} is "γ > R_{SD} > β" and there is "body movement" (Step S110: Yes, S112), or if it is determined that the value of R_{SD} is not "γ > R_{SD} > β" (Step S110: No), it proceeds to the step of determining whether or not " R_{SD} > γ".

Next, when it is determined that the value of R_{SD} is "R_{SD} > γ" and "electrode non-contact" (Step S114: Yes, S116) or not "R_{SD} > γ" (Step S114: No), it proceeds to the step of determining whether it is "measurement abnormality" or not.

Finally, when the body composition analyzer 10 determines that is not "dry," "body movement," or "electrode non-contact" and therefore is not "measurement abnormal" (step S118: No), the weight and body composition data are acquired and displayed (step S120), and the flow ends. On the other hand, when the body composition analyzer 10 determines that is at least one of "dry," "body movement," and "electrode non-contact" (Step S118: Yes), the cause of the abnormality and remedial measures are notified (Step S122), and the flow returns to Step S102.

Thus, according to the first embodiment, the cause or type of abnormality of the measurement can be determined based on the time-series change of the measured bioelectrical impedance. Therefore, measurement abnormality can be determined without having to provide a circuit for detecting measurement abnormality separately from the circuit for measuring bioelectrical impedance.

According to the first embodiment, measurement abnormalities can be determined with high accuracy by using trends (trend variation) such as the slope of a linear function when time-series data is approximated by a linear function, and variations such as the standard deviation, variance, and unbiased variance of time-series data.

Furthermore, according to the first embodiment, the user can know the cause or type of measurement abnormality, and can know improvement measures for normal measurement corresponding to the cause or type of measurement abnormality.

### (Second Embodiment)

The body composition analyzer 10 of the second embodiment has the same basic configuration as the body composition analyzer 10 of the first embodiment. The difference is that in the first embodiment, the body composition analyzer 10 determines whether or not there is a measurement abnormality based on the parameter pertaining to the resistance (R), whereas in the second embodiment, the body composition analyzer 10 determines whether or not there is a measurement abnormality based on the parameter pertaining to the resistance (R) and the inductance (X) . In the following, this difference and the operation flow of the body composition analyzer will be explained.

### [Composition of Body Composition Analyzer 10]

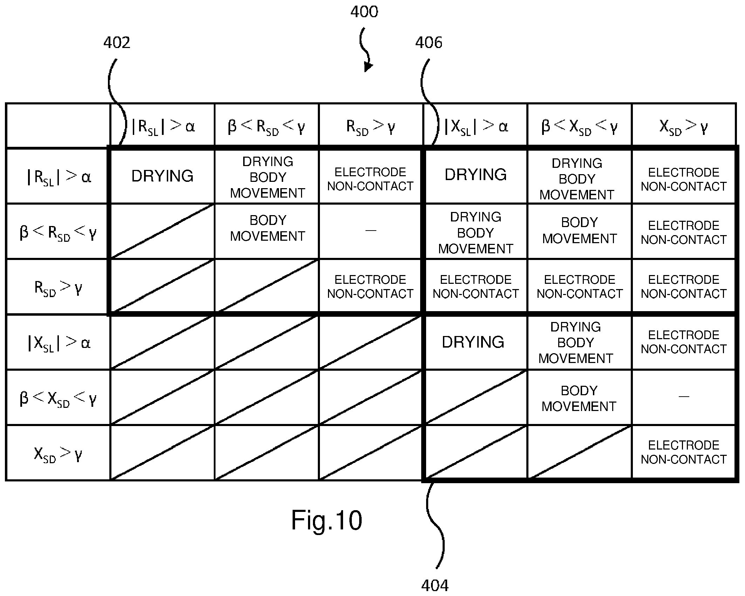

As shown in Fig. 10, the measurement abnormality determination unit 116 has a table 400 indicating the relationship between the following (a) and (b): (a)the absolute value (|R_{SL}|) of the slope of the linear function and the standard deviation (R_{SD}) when the time-series data of R is approximated by a linear function and the absolute value (|X_{SL}|) of the slope of the linear function and the standard deviation (X_{SD}) when the time-series data of X is approximated by a linear function, and (b) the situations of electrode non-contact, drying, and body movement.

The table 400 has three sub-tables. Of these, the sub-table 402 is the same as the table 200 of the first embodiment, and the sub-table 404 is a table in which the |R_{SL}| > α, β < R_{SD} < γ, and R_{SD} > γ of the table 200 are changed to |X_{SL}| > α, β < X_{SD} < γ, and X_{SD} > γ, respectively, and thus is not described.

The sub-table 406 is a table indicating that when "R_{SD} > γ" or "X_{SD} > γ", it is determined that the electrode and the living body are not in correct contact compared to the normal measurement. When "|R_{SL}| > α" or "|X_{SL}| > α" is selected, the table indicates that the skin is dry or socks are worn, compared to normal measurement. The table also indicates that when "β < R_{SD} < γ" or "β < X_{SD} < γ", compared to the normal measurement, it is determined that there is body movement.

The measurement abnormality determination unit 116 refers to this table 400 and determines whether the measurement is abnormal or not and the type of abnormality when the measurement is abnormal based on the value of the parameter |R_{SL}|, the value of R_{SD}, the value of |X_{SL}|, and the value of X_{SD} generated by the parameter value generating unit 114.

### [Operation Flow of the Body Composition Analyzer 10]

The following describes a flow for realizing the operation of the body composition analyzer 10 of the second embodiment by the above-described configuration of the body composition analyzer 10.

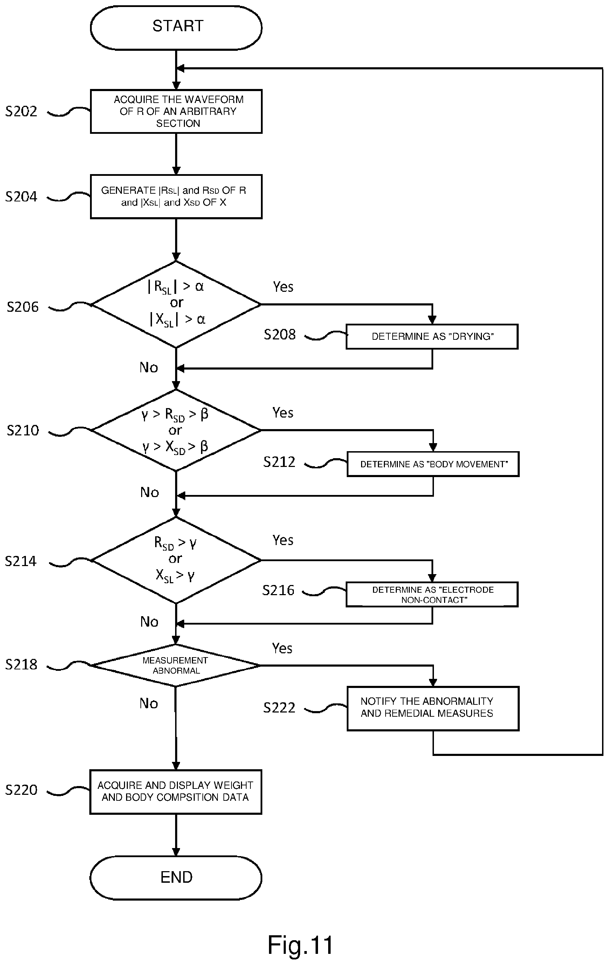

Fig. 11 shows a flow chart of the second embodiment, for determining a measurement abnormality based on the absolute value (|R_{SL}|) of the slope of the linear function and the standard deviation (R_{SD}) when the time-series data of R is approximated by a linear function and the absolute value (|X_{SL}|) of the slope of the linear function and the standard deviation (X_{SD}) when the time-series data of X is approximated by a linear function. When a user is on the body composition analyzer 10, the flow starts.

First, the body composition analyzer 10 acquires the waveform of R and X in an arbitrary section (step S202).

When the body composition analyzer 10 acquires the waveform of R and X in an arbitrary section, it generates the value of |R_{SL}|, the value of R_{SD}, the value of |X_{SL}|, and the value of X_{SD} (Step S204) . Then, the body composition analyzer 10 determines whether these values are "|R_{SL}| > α or |X_{SL}| >α" (Step S206), "γ > R_{SD} > β or γ > X_{SD} > β" (Step S210), and "R_{SD} > γ or X_{SD} > γ" (Step S214), respectively.

First of all, when it is determined that the value of "|R_{SL}|" and the value of "|X_{SL}|" are "|R_{SL}| > α or |X_{SL}|> α" and "drying" (Step S206: Yes, S208), or when it is determined that it is not "|R_{SL}| > α or |X_{SL}| > α" (Step S206: No), it proceeds to the step of determining whether "γ > R_{SD} > β or γ > X_{SD} > β".

Next, when it is determined that the value of R_{SD} and the value of X_{SD} are "γ > R_{SD} > β or γ > X_{SD} > β" and there is "body movement" (Step S210: Yes, S212), when it is determined that it is not "γ > R_{SD} > β or γ > X_{SD} > β" (Step S210: No), it proceeds to the step of determining whether "R_{SD} > γ or X_{SD} > γ".

Next, when it is determined that the value of R_{SD} and the value of X_{SD} are "R_{SD} > γ or X_{SD} > γ" and "electrode non-contact" (Step S214: Yes, S216) or not "R_{SD} > γ or X_{SD} > γ" (Step S214: No), it proceeds to the step of determining whether or not it is "measurement abnormality".

Finally, when the body composition analyzer 10 determines that it is not "drying," "with body movement," or "without electrode contact" and therefore is not a "measurement abnormality" (Step S218: No), the weight and body composition data are acquired and displayed (Step S220), and the flow ends. On the other hand, when the body composition analyzer 10 determines that is at least one of "drying," "body movement," and "electrode non-contact" (Step S218: Yes), the cause of the abnormality and remedial measures are notified (Step S222), and the flow returns to Step S202.

Thus, according to the second embodiment, the measurement abnormality can be determined more accurately based on the resistance (R), which is mainly derived electrically from the extracellular fluid, and the reactance (X), which is mainly derived electrically from the intracellular fluid and the cell membrane.

### [Variant 1]

In the first embodiment, the body composition analyzer 10 determines a measurement abnormality using a parameter pertaining to R. However, a measurement abnormality may be determined using a parameter pertaining to X. That is, the sub-table 404 may be used to determine the measurement abnormality.

### [Variant 2]

In the second embodiment, for example, "|R_{SL}| > α or |X_{SL}| > α" is used to determine "drying" if at least one of "|R_{SL}| > α" or "|X_{SL}| > α" is satisfied, but "drying" may be determined if both "|R_{SL}| > α" and "|X_{SL}| > α" are satisfied. In other words, it may be determined as "drying" when "|R_{SL}| > α and |X_{SL}| > α" is satisfied. Similarly, it may be determined as "body movement" when "γ > R_{SD} > β and γ > X_{SD} > β" is satisfied, and it may be may be as "electrode non-contact" when "R_{SD} > γ and X_{SD} > γ" is satisfied.

### [Variant 3]

In the second embodiment, for example, the same threshold value α was used as "|R_{SL}| > α or |X_{SL}| > α", but a different threshold value α and δ may be used as "|R_{SL}| > α or |X_{SL}| > δ". Similarly, different threshold values β and ε, γ and ζ can be used to make "γ > R_{SD} > β or ζ > X_{SD} > ε" or "R_{SD} > γ or X_{SD} > ζ".

### [Variant 4]

When the process of notifying the cause of abnormality and remedial measures in Fig. 9 or Fig. 11 is performed a predetermined number of times (e.g., five times), the body composition data may be calculated with the impedance even if the next measurement abnormality is determined. Also, when the notification process has been performed a predetermined number of times with the same "cause of abnormality and remedial measures," the body composition data may be calculated with that impedance even if the measurement abnormality is determined to be caused by the same cause of abnormality next. In these cases, the displayed body composition data may be displayed together with the fact that it was an impedance measurement abnormality, or in addition to this, the cause of the impedance measurement abnormality.

### DESCRIPTION OF THE CODE

- 10: Body composition analyzer
- 20: Main unit
- 22L, R: Current-carrying electrode
- 24L, R: Measuring electrode
- 102: Input unit
- 104: Memory unit
- 106: Output unit
- 108: Control unit
- 110: Measuring unit
- 112: Bioelectrical impedance measuring unit
- 114: Parameter value generation unit
- 116: Measurement abnormality determination unit
- 118: Notification unit
- 120: Body composition data acquisition unit
- 200: Table
- 400: Table

## Claims

1. A body composition analyzer for measuring body composition based on the measurement of bioelectrical impedance, comprising:
a bioelectrical impedance measuring unit configured to acquire time-series data of bioelectrical impedance by measurement, and
a measurement abnormality determination unit configured to determine a cause or type of abnormality in the measurement based on the time-series data.

2. The body composition analyzer according to claim 1, wherein the measurement abnormality determining unit is configured to determine the cause or type of the abnormality of the measurement based on a trend of the time-series data.

3. The body composition analyzer according to claim 1 or 2, wherein the measurement abnormality determination unit is configured to determine the cause or type of the abnormality of the measurement based on the variation of the time-series data.

4. The body composition analyzer according to any of claims 1 to 3, wherein the bioelectrical impedance includes resistance.

5. The body composition analyzer according to any of claims 1 to 4, wherein the bioelectrical impedance includes reactance.

6. The body composition analyzer according to any of claims 1 to 5, further comprising a notification unit configured to notify a remedial measure corresponding to the cause or type of the abnormality in the measurement.

7. A body composition measurement program for controlling a body composition analyzer equipped with a computer for measuring body composition based on measurement of bioelectrical impedance, causing the computer to:
acquire time-series data of the bioelectrical impedance by measurement; and
determine a cause or type of abnormality in the measurement based on the time-series data.

8. A computer-readable non-transitory storage medium storing a body composition measurement program for controlling a body composition analyzer equipped with a computer for measuring body composition based on measurement of bioelectrical impedance, the program causing the computer to:
acquire time-series data of the bioelectrical impedance by measurement; and
determine a cause or type of abnormality in the measurement based on the time-series data.
